# EUROPEAN PATENT APPLICATION

(11) **EP 4 834 886 A1**
(43) Date of publication of application: **04.11.2026**
(21) Application number: 25173197.2
(22) Date of filing: 29.04.2025
(51) Int. Cl.: C07D 239/54, A01N 43/54

(54) **CHLORODEAMINATION REACTION FOR THE PREPARATION OF SUBSTITUTED N-PHENYLURACILS**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to processes for the synthesis of substituted N-phenyluracils of formula (I).

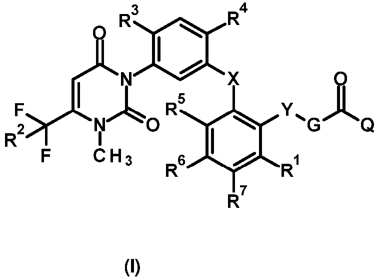

## Description

The present invention relates to processes for the synthesis of substituted N-phenyluracils of formula (I).

Substituted N-phenyluracils are known in the art, *see* for example WO2024/078906. Due to the various and partly reactive side groups of such compounds, their synthesis is challenging. In particular, N-phenyluracils as described in the literature comprise an aryl chloride in their core structure. Such aryl chloride may be accessed from the corresponding aniline structure via a diazonium salt in a Sandmeyer reaction. However, isolating or cumulating such a high-energy diazonium intermediate can be highly dangerous in terms of energetic potential. In addition, such transformations typically provide only moderate yields.

Science 2024, 384, 446 describes a process for the conversion of anilines into aryl halides.

J. Org. Chem. 1977, 42, 2426 describes a process using organic alkyl nitrites and copper(II) chloride to transform anilines to the corresponding aryl chloride.

WO2014142307 A1 *inter alia* describes the conversion of an aniline structure to a halogen-substituted structure in a Sandmeyer reaction.

It has now been found that most reaction conditions described in the art and tested for the Sandmeyer conversion of anilines into structures of formula (I) give rise to significant amounts of hydrodeaminated aniline as unwanted side product. In addition, varying amounts of undefined polymers arise from the reaction. Furthermore, high amounts of copper salt waste arise. In addition, the starting aniline structure of formula (II) already displays a high decomposition energy, even before being converted to the energy-rich diazonium functional group, requiring very selective reaction conditions for this educt. This highlights the importance of careful reaction kinetics control.

At least parts of these problems have been solved by the present invention.

It has been found that specific process parameters allow for a high yield of product according to formula (I), using an equimolar amount of copper(I) and copper(II) salts, while preventing the buildup of high energy-intermediates.

The present invention thus relates to a chlorodeamination reaction, also known as Sandmeyer reaction for the preparation of compounds of formula **(I)** 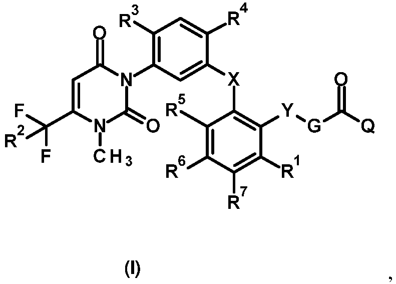 wherein
- R¹: is hydrogen, fluorine, chlorine or bromine,
- R²: is fluorine,
- R³: is fluorine,
- R⁴: is chlorine,
- R⁵: is hydrogen, fluorine, chlorine or bromine,
- R⁶: is hydrogen, fluorine, chlorine, bromine or cyano,
- R⁷: is hydrogen, fluorine, chlorine or bromine,
- G: is methylene, (methyl)methylene or (ethyl)methylene,
- X and: Y are independently O (oxygen) or S (sulfur)
and
- Q: is hydroxy or one of the moieties Q-1 to Q-25 specifically mentioned below, where the arrow in the structural formulae in the table below represents a bond of the respective Q group to carbonyl group in the general formula (I),

| | | | | |
|---|---|---|---|---|
| 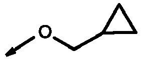 | 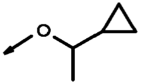 | 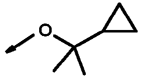 | 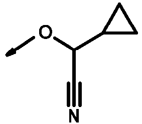 | 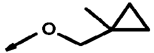 |
| Q-1 | Q-2 | Q-3 | Q-4 | Q-5 |

| | | | | |
|---|---|---|---|---|
| 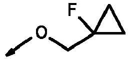 | 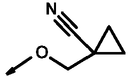 | 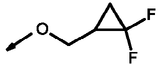 | 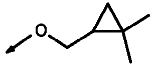 | 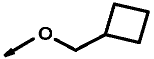 |
| Q-6 | Q-7 | Q-8 | Q-9 | Q-10 |

| | | | | |
|---|---|---|---|---|
| 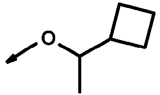 | 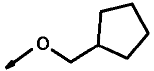 | 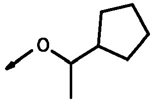 | 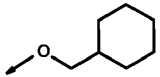 | 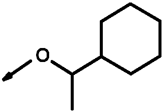 |
| Q-11 | Q-12 | Q-13 | Q-14 | Q-15 |

| | | | | |
|---|---|---|---|---|
| 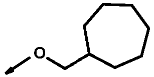 | 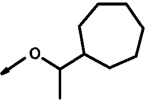 | 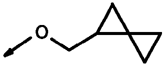 | 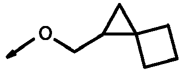 | 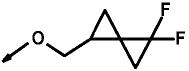 |
| Q-16 | Q-17 | Q-18 | Q-19 | Q-20 |

| | | | | |
|---|---|---|---|---|
| 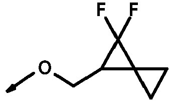 | 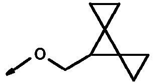 | 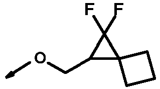 | 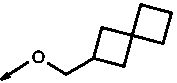 | 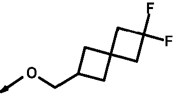 |
| Q-21 | Q-22 | Q-23 | Q-24 | Q-25 |

wherein compounds of formula **(II)** 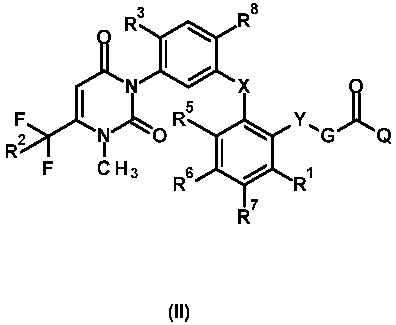 wherein R⁸ is NH₂,
are reacted in the presence of an organic solvent, an inorganic or organic nitrite, an inorganic acid and a catalyst comprising copper (I) chloride and copper (II) chloride.

Preferably, in compounds of formula (I) and (II),
- R¹: is hydrogen,
- R²: is fluorine,
- R³: is fluorine,
- R⁵: is hydrogen,
- R⁶: is hydrogen, fluorine, chlorine,
- R⁷: is hydrogen,
- G: is methylene, (methyl)methylene,
- X: is O (oxygen) or S (sulfur),
- Y: is O (oxygen)
and
- Q: is hydroxy or one of the moieties Q-1 to Q-25 specifically mentioned above.

Further preferably, in compounds of formula (I) and (II),
- R¹: is hydrogen,
- R²: is fluorine,
- R³: is fluorine,
- R⁵: is hydrogen,
- R⁶: is hydrogen or fluorine,
- R⁷: is hydrogen,
- G: is methylene,
- X: is O (oxygen),
- Y: is O (oxygen)
and
- Q: is hydroxy or represents one of the moieties Q-1, Q-2, Q-4, Q-5. Q-6, Q-7, Q-8, Q-9, Q-10, Q-12, Q-13, Q-14, Q-15, Q-16, Q-18 or Q-19.

Even further preferably, in compounds of formula (I) and (II),
- R¹: is hydrogen,
- R²: is fluorine,
- R³: is fluorine,
- R⁵: is hydrogen,
- R⁶: is hydrogen,
- R⁷: is hydrogen,
- G: is methylene,
- X: is O (oxygen),
- Y: is O (oxygen)
and
- Q: is hydroxy or Q-1.

Preferably, the organic solvent is selected from alcoholic solvents, such as tert-butanol and ethanol, carbonates, such as dimethyl carbonate, or nitrogen containing organic solvents, such as acetonitrile.

Further preferably, the organic solvent is acetonitrile.

Preferably, the inorganic acid is HCl.

The inorganic acid may be provided in a form suitable for a scale-up production, such as in gaseous form or dissolved, e.g. gaseous HCl or HCl provided in acetic acid.

The inorganic nitrite may be selected from alkali metal nitrites, such as sodium or potassium nitrite, preferably sodium nitrite.

The organic nitrite may be an alkyl nitrite, preferably selected from ethyl nitrite, propyl nitrite, isopropyl nitrite, n-butyl nitrite, tert-butyl nitrite, n-pentyl nitrite, or mixtures thereof. Further preferably, the organic nitrite is tert-butyl nitrite.

Most preferably, the nitrite compound is tert-butyl nitrite.

The reaction may be performed at a temperature of between -10 °C and 80 °C, preferably between 0 °C and 30 °C, further preferably between 15 °C and 25 °C.

After the reaction, the compound of formula (I) may be purified by means of aqueous work up and crystallization.

It has been found that the reaction conditions described herewith allow for a relatively low amount of copper chloride salts to be used. Specifically, it is sufficient to apply at most 1.2 eq., preferably at most 1.1 eq., even further preferably at most about 1 eq. CuCl to the compound of formula (II). It is furthermore sufficient to apply at most 1.2 eq., preferably at most 1.1 eq., even further preferably at most about 1 eq. CuCl₂ to the compound of formula (II).

It is furthermore sufficient to apply at most 1.5 eq., preferably at most 1.4 eq., even further preferably at most about 1.1 eq. of the inorganic or organic nitrite, preferably of the alkyl nitrite, and even further preferably of tert-butyl nitrite to the compound of formula (II).

A further aspect of the present invention relates to compound of formula (II). These structures are valuable intermediates in the synthesis of agrochemical compounds.

### Examples

The following examples starting from the aniline compound A (cyclopropylmethyl 2-[2-[2-amino-4-fluoro-5-[3-methyl-2,6-dioxo-4-(trifluoromethyl)pyrimidin-1-yl]phenoxy]phenoxy]acetate) illustrate the invention further but do not intend to limit it.

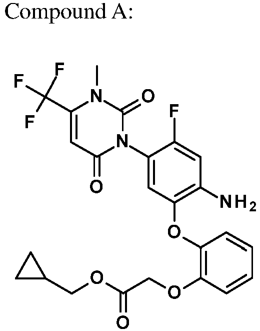

### Example 1

Hydrochloric acid 1N in acetic acid (8.74 mL, 8.74 mmol, 1.0 eq.) was slowly added to a stirred solution of aniline A (4,57 g, 8.74 mmol) in acetonitrile (20 mL). Cupric chloride (1.17 g. 8.74 mmol, 1.0 eq.) was then added, followed by cuprous chloride (0.86 g. 8.74 mmol, 1.0 eq.). *tert-*Butyl nitrite (0.99 g, 9.61 mmol, 1.1 eq.) was then added via syringe pump over 15 min and the reaction mixture was allowed to stir for another 30 minutes at room temperature (approx. 21 °C). At this point, all the starting aniline is consumed. The reaction is quenched with sulfamic acid (0.59 g, 6.12 mmol, 0.7 eq.), stirred for a further 30 min and filtered over celite. The filter cake was washed with acetonitrile. Analysis of the filtrate by quantitative HPLC revealed a yield of 92%. The volatiles of the filtrate were distilled off under reduced pressure and the mixture was taken up in *tert-*amyl methyl ether (30 mL) and washed once with a 10% hydrochloric aqueous solution, twice with a saturated ammonium chloride solution and once with brine. The resulting organic phase was dried over magnesium sulfate, filtered and evaporated under reduced pressure. The residue was taken up in 15 mL of a 1:1 mixture of *tert*-amyl methyl ether and methylcyclohexane. The mixture was heated to 50 °C, then slowly cooled to 0 °C. The product was isolated by filtration (3.4 g, 94% purity).

### Example 2

Hydrochloric acid 1N in acetic acid (18.1 mL, 18.1 mmol, 1.0 eq.) was slowly added to a stirred solution of aniline A (9.47 g, 18.1 mmol) in acetonitrile (40 mL). Cupric chloride (2.43 g. 18.1 mmol, 1.0 eq.) was then added, followed by cuprous chloride (1.79 g. 18.1 mmol, 1.0 eq.). *tert-*Butyl nitrite (2.05 g, 19.9 mmol, 1.1 eq.) was then added via syringe pump over 15 min and the reaction mixture was allowed to stir for another 30 minutes at room temperature. At this point, all the starting aniline is consumed. The reaction is quenched with sulfamic acid (0.53 g, 5.43 mmol, 0.3 eq.), stirred for a further 30 min and filtered over celite. The filter cake was washed with acetonitrile. Analysis of the filtrate by quantitative HPLC revealed a yield of 88%. The volatiles of the filtrate were distilled off under reduced pressure and the mixture was taken up in cyclopentyl methyl ether (70 mL) and washed once with a 17% aqueous ammonia solution and once with a saturated ammonium chloride solution. The resulting organic phase was dried over magnesium sulfate, filtered and evaporated under reduced pressure. The residue was taken up in 30 mL of a 1:1 mixture of diisopropyl ketone and methylcyclohexane. The mixture was heated to 80 °C, then slowly cooled to 10°C. The product was isolated by filtration (7.4 g, 97% purity, 73% yield).

### Example 3

The hydrochloride salt of the aniline A (190 mg, 0.34 mmol) was suspended in ethyl acetate (22 mL). Cupric chloride (46 mg. 0.34 mmol, 1.0 eq.) was then added, followed by cuprous chloride (7 mg. 0.07 mmol, 0.2 eq.). *tert-*Butyl nitrite (72 µL of a 90% solution, 0.55 mmol, 1.62 eq.) was then added via syringe and the reaction mixture was allowed to stir for two hours at room temperature. The reaction mixture was diluted with acetonitrile (20 mL) and the solution was analyzed by quantitative HPLC. The yield obtained is 42%.

### Example 4

The hydrochloride salt of the aniline A (190 mg, 0.34 mmol) was suspended in acetonitrile (22 mL). Cupric chloride (46 mg. 0.34 mmol, 1.0 eq.) was then added, followed by cuprous chloride (7 mg. 0.07 mmol, 0.2 eq.). *tert-*Butyl nitrite (72 µL of a 90% solution, 0.55 mmol, 1.62 eq.) was then added via syringe and the reaction mixture was allowed to stir for two hours at room temperature. The reaction mixture was diluted with acetonitrile (20 mL) and the solution was analyzed by quantitative HPLC. The yield obtained is 73%.

### Example 5

The hydrochloride salt of the aniline A (190 mg, 0.34 mmol) was suspended in dichloromethane (22 mL). Cupric chloride (46 mg. 0.34 mmol, 1.0 eq.) was then added, followed by cuprous chloride (7 mg. 0.07 mmol, 0.2 eq.). *tert-*Butyl nitrite (72 µL of a 90% solution, 0.55 mmol, 1.62 eq.) was then added via syringe and the reaction mixture was allowed to stir for two hours at room temperature. The reaction mixture was diluted with acetonitrile (20 mL) and the solution was analyzed by quantitative HPLC. The yield obtained is 32%.

### Example 6

The hydrochloride salt of the aniline A (190 mg, 0.34 mmol) was suspended in a 9:1 mixture of acetonitrile : water (22 mL). Cupric chloride (46 mg. 0.34 mmol, 1.0 eq.) was then added, followed by cuprous chloride (7 mg. 0.07 mmol, 0.2 eq.). *tert-*Butyl nitrite (72 µL of a 90% solution, 0.55 mmol, 1.62 eq.) was then added via syringe and the reaction mixture was allowed to stir for two hours at room temperature. The reaction mixture was diluted with acetonitrile (20 mL) and the solution was analyzed by quantitative HPLC. The yield obtained is 54%.

### Example 7

The hydrochloride salt of the aniline A (190 mg, 0.34 mmol) was suspended in acetonitrile (22 mL). Cupric chloride (135 mg. 1.01 mmol, 3.0 eq.) was then added, followed by cuprous chloride (100 mg. 1.01 mmol, 3.0 eq.). *tert-*Butyl nitrite (72 µL of a 90% solution, 0.55 mmol, 1.62 eq.) was then added via syringe and the reaction mixture was allowed to stir for two hours at room temperature. The reaction mixture was diluted with acetonitrile (20 mL) and the solution was analyzed by quantitative HPLC. The yield obtained is 91%.

### Example 8

Hydrochloric acid 1N in acetic acid (8.74 mL, 8.74 mmol, 1.0 eq.) was slowly added to a stirred solution of aniline A (4.57 g, 8.74 mmol) in acetonitrile (30 mL). Cupric chloride (1.17 g. 8.74 mmol, 1.0 eq.) was then added, followed by cuprous chloride (865 mg. 8.74 mmol, 1.0 eq.). *tert-*Butyl nitrite (990 mg, 9.61 mmol, 1.1 eq.) was then added via syringe and the reaction mixture was allowed to stir for another 90 minutes at room temperature. At this point, all the starting aniline is consumed. The reaction is quenched with sulfamic acid (594 mg, 6.12 mmol, 0.7 eq.), stirred for a further 30 min and filtered over celite. The filter cake was washed with acetonitrile. Analysis of the filtrate by quantitative HPLC revealed a yield of 96%. The volatiles of the filtrate were evaporated under reduced pressure and the mixture was taken up methyl *tert-*butyl ether and washed once with a 10% HCl aqueous solution, twice with a saturated ammonium chloride solution and once with brine. The resulting organic phase was dried over magnesium sulfate, filtered and evaporated under reduced pressure. The residue was taken up in 15 mL of diisopropylether and stirred over 2 days at room temperature. The crystallized product was collected by filtration, washing with ice cold methyl *tert-*butyl ether (3.1 g, 97.2% purity, 64% yield).

The following example is starting from the aniline compound B.

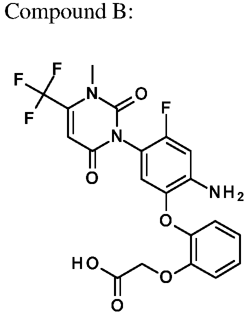

### Example 9

Hydrochloric acid 1N in acetic acid (7.6 mL, 7.6 mmol, 0.5 eq.) was slowly added to a stirred solution of aniline B (7.13 g, 15.2 mmol) in acetonitrile (63 mL). Cupric chloride (2.04 g. 15.2 mmol, 1.0 eq.) was then added, followed by cuprous chloride (1.50 g. 15.2 mmol, 1.0 eq.). *tert-*Butyl nitrite (1.72 g, 16.7 mmol, 1.1 eq.) was then added via syringe pump over 15 min and the reaction mixture was allowed to stir for another 30 minutes at room temperature. At this point, all the starting aniline is consumed. The reaction is quenched with sulfamic acid (0.44 g, 4.56 mmol, 0.3 eq.), stirred for a further 30 min and filtered over celite. The filter cake was washed with acetonitrile. Analysis of the filtrate by quantitative HPLC revealed a yield of 88%.

## Claims

1. Chlorodeamination reaction for the preparation of compounds of formula **(I)** 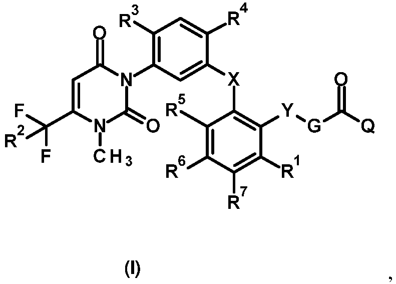 wherein
R¹ is hydrogen, fluorine, chlorine or bromine,
R² is fluorine,
R³ is fluorine,
R⁴ is chlorine,
R⁵ is hydrogen, fluorine, chlorine or bromine,
R⁶ is hydrogen, fluorine, chlorine, bromine or cyano,
R⁷ is hydrogen, fluorine, chlorine or bromine,
G is methylene, (methyl)methylene or (ethyl)methylene,
X and Y are independently O (oxygen) or S (sulfur) and
Q is hydroxy or one of the moieties Q-1 to Q-25 specifically mentioned below, where the arrow in the structural formulae in the table below represents a bond of the respective Q group to carbonyl group in the general formula (I),
| | | | | |
|---|---|---|---|---|
| 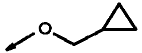 | 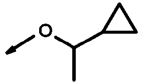 | 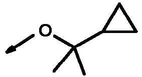 | 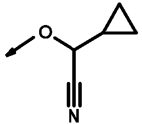 | 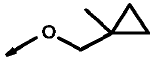 |
| Q-1 | Q-2 | Q-3 | Q-4 | Q-5 |
| | | | | |
|---|---|---|---|---|
| 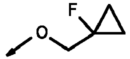 | 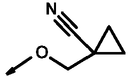 | 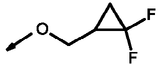 | 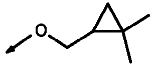 | 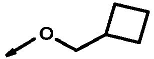 |
| Q-6 | Q-7 | Q-8 | Q-9 | Q-10 |
| | | | | |
|---|---|---|---|---|
| 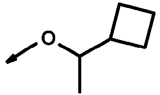 | 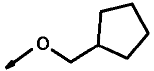 | 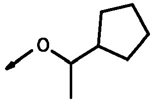 | 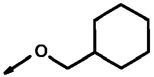 | 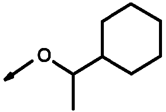 |
| Q-11 | Q-12 | Q-13 | Q-14 | Q-15 |
| | | | | |
|---|---|---|---|---|
| 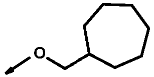 | 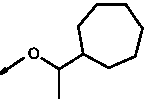 | 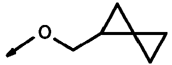 | 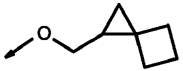 | 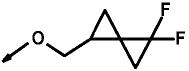 |
| Q-16 | Q-17 | Q-18 | Q-19 | Q-20 |
| | | | | |
|---|---|---|---|---|
| 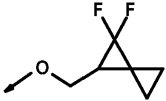 | 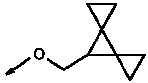 | 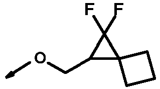 | 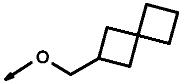 | 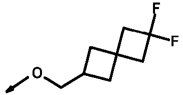 |
| Q-21 | Q-22 | Q-23 | Q-24 | Q-25 |
wherein compounds of formula **(II)** 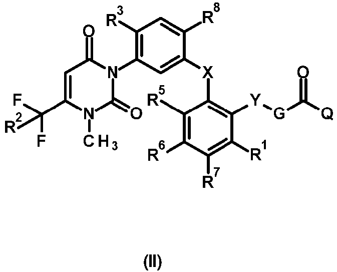 wherein R⁸ is NH₂,
are reacted in the presence of an organic solvent, an inorganic or organic nitrite, an inorganic acid and a catalyst comprising copper (I) chloride and copper (II) chloride.

2. Process according to claim 1, wherein
R¹ is hydrogen,
R² is fluorine,
R³ is fluorine,
R⁵ is hydrogen,
R⁶ is hydrogen, fluorine, chlorine,
R⁷ is hydrogen,
G is methylene, (methyl)methylene,
X is O (oxygen) or S (sulfur),
Y is O (oxygen) and
Q is hydroxy or one of the moieties Q-1 to Q-25 specifically mentioned above.

3. Process according to claim 1, wherein
R¹ is hydrogen,
R² is fluorine,
R³ is fluorine,
R⁵ is hydrogen,
R⁶ is hydrogen or fluorine,
R⁷ is hydrogen,
G is methylene,
X is O (oxygen),
Y is O (oxygen) and
Q is hydroxy or one of the moieties Q-1, Q-2, Q-4, Q-5. Q-6, Q-7, Q-8, Q-9, Q-10, Q-12, Q-13, Q-14, Q-15, Q-16, Q-18 or Q-19.

4. Process according to claim 1, wherein
R¹ is hydrogen,
R² is fluorine,
R³ is fluorine,
R⁵ is hydrogen,
R⁶ is hydrogen,
R⁷ is hydrogen,
G is methylene,
X is O (oxygen),
Y is O (oxygen) and
Q is hydroxy or Q-1.

5. Process according to any one of the preceding claims, wherein the organic solvent is selected from alcoholic solvents, such as *tert*-butanol and ethanol, carbonates, such as dimethyl carbonate, or nitrogen containing organic solvents, such as acetonitrile.

6. Process according to any one of the preceding claims, wherein the organic solvent is acetonitrile.

7. Process according to any one of the preceding claims, wherein the inorganic acid is HCl.

8. Process according to any one of the preceding claims, wherein the organic nitrite is an alkyl nitrite, preferably selected from ethyl nitrite, propyl nitrite, isopropyl nitrite, n-butyl nitrite, tert-butyl nitrite, n-pentyl nitrite, or mixtures thereof; or wherein the inorganic nitrite is an alkali metal nitrite, preferably sodium nitrite or potassium nitrite.

9. Process according to any one of the preceding claims, wherein the organic nitrite is tert-butyl nitrite.

10. Process according to any one of the preceding claims, wherein reaction is performed at a temperature of between -10 °C and 80 °C, preferably between 0 °C and 30 °C, further preferably between 15 °C and 25 °C.

11. Process according to any one of the preceding claims, wherein after the reaction, the compound of formula (I) is purified by means of crystallization.

12. Process according to any one of the preceding claims, wherein at most 1.2 eq., preferably at most 1.1 eq., even further preferably at most about 1 eq. copper(I) chloride is applied, relative to the compound of formula (II).

13. Process according to any one of the preceding claims, wherein at most 1.2 eq., preferably at most 1.1 eq., even further preferably at most about 1 eq. copper(II) chloride is applied, relative to the compound of formula (II).

14. Process according to any one of the preceding claims, wherein at most 1.5 eq., preferably at most 1.4 eq., even further preferably at most about 1.1 eq. of the organic nitrite, preferably tert-butyl nitrite is applied, relative to the compound of formula (II).
